(19) Europäisches Patentamt
European Patent Office
Office européen des brevets

(11) EP 0 983 994 A2

# (12) EUROPÄISCHE PATENTANMELDUNG

(43) Veröffentlichungstag:
**08.03.2000 Patentblatt 2000/10**

(51) Int. Cl.$^7$: **C07C 211/28**, C07C 215/54, C07C 217/62

(21) Anmeldenummer: **99119108.1**

(22) Anmeldetag: **22.02.1997**

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **13.03.1996 DE 19609847**

(62) Dokumentnummer(n) der früheren Anmeldung(en) nach Art. 76 EPÜ:
**97102923.6 / 0 799 819**

(71) Anmelder: **Grünenthal GmbH**
**52078 Aachen (DE)**

(72) Erfinder:
- **Buschmann, Helmut Heinrich, Dr.**
  **52066 Aachen (DE)**
- **Strassburger, Wolfgang Werner Alfred, Prof. Dr.**
  **52146 Würselen (DE)**
- **Koegel, Babette-Yvonne, Dr.**
  **52379 Lanerwehe-Hamich (DE)**
- **Friedrichs, Elmar Josef, Dr.**
  **52223 Stolberg (DE)**

Bemerkungen:
Diese Anmeldung ist am 04 - 10 - 1999 als Teilanmeldung zu der unter INID-Kode 62 erwähnten Anmeldung eingereicht worden.

## (54) Dimethyl-(3-aryl-but-3-enyl)-aminverbindungen als pharmazeutische Wirkstoffe

(57) Es werden Dimethyl-(3-aryl-but-3-enyl)-aminverbindungen, Verfahren zu deren Herstellung sowie die Verwendung dieser Verbindungen als pharmazeutische Wirkstoffe beschrieben.



Printed by Xerox (UK) Business Services
2.16.7/3.6

**Beschreibung**

Die Erfindung betrifft Dimethyl-(3-aryl-but-3-enyl)-aminverbindungen, Verfahren zu deren Herstellung sowie die Verwendung dieser Verbindungen in Arzneimitteln.

Die Behandlung chronischer und nichtchronischer Schmerzzustände hat in der Medizin eine große Bedeutung. Zur Zeit besteht ein weltweiter Bedarf an zusätzlicher, nicht ausschließlich opioider, aber gut wirksamer Schmerztherapie. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik sowie der Grundlagenforschung zur Nociception in letzter Zeit erschienen sind.

Opioide werden seit vielen Jahren als Analgetika zur Schmerzbehandlung eingesetzt, obwohl sie eine Reihe von Nebenwirkungen, beispielsweise Sucht und Abhängigkeit, Atemdepression, gastro-intestinale Hemmwirkung und Obstipation, hervorrufen. Sie können daher nur unter besonderen Vorsichtsmaßnahmen wie z.B. speziellen Verordnungsvorschriften über einen längeren Zeitraum oder in höheren Dosierungen gegeben werden (Goodman, Gilman, The Pharmacological Basis of Therapeutics, Pergamon Press, New York 1990).

Tramadolhydrochlorid - (1RS, 2RS)-2-Dimethylaminomethyl-1-(3-methoxyphenyl)-cyclohexanol, Hydrochlorid - nimmt unter den zentralwirksamen Analgetika eine Sonderstellung ein, da dieser Wirkstoff eine starke Schmerzhemmung ohne die für Opioide bekannten Nebenwirkungen hervorruft (J. Pharmacol. Exptl. Ther. 267 331 (1993)). Tramadol ist ein Racemat und besteht aus gleichen Mengen an (+)- und (-)-Enantiomer. In vivo bildet der Wirkstoff den Metaboliten O-Desmethyl-tramadol, der gleichfalls als Enantiomerengemisch vorliegt. Untersuchungen haben ergeben, daß sowohl die Enantiomeren von Tramadol als auch die Enantiomeren der Tramadolmetabolite an der analgetischen Wirkung beteiligt sind (J. Pharmacol. Exptl. Ther. 260 275 (1992)).

Die der Erfindung zugrundeliegende Aufgabe bestand in der Entwicklung von analgetisch wirksamen Substanzen, die sich zur Behandlung starker Schmerzen eignen, ohne die für Opioide typischen Nebenwirkungen hervorzurufen. Darüber hinaus sollten die zu entwickelnden Substanzen nicht die wahrend der Behandlung mit Tramadol in manchen Fällen auftretenden Nebenwirkungen, beispielsweise Übelkeit und Erbrechen, besitzen.

Es wurde gefunden, daß die an die zu entwikkelnden Substanzen gestellten Anforderungen von bestimmten Dimethyl-(3-aryl-but-3-enyl)-aminen erfüllt werden. Diese Substanzen zeichnen sich durch eine ausgeprägte analgetische Wirkung aus, die im Vergleich zu Tramadol deutlich verstärkt ist

Gegenstand der Erfindung sind dementsprechend Dimethyl-(3-aryl-but-3-enyl)-aminverbindungen der Formel I

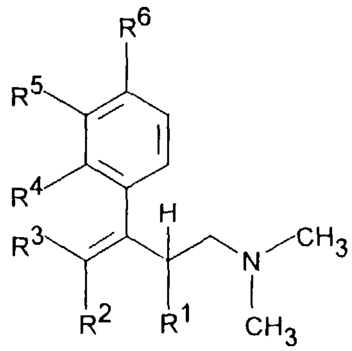

in der $R^1$ und $R^2$ zusammen $-(CH_2)_{2-4}-$, $-(CH_2)_2-CHR^7$ oder $-CH_2-CHR^7-CH_2-$ darstellen, $R^3$ H oder $C_{1-5}$-Alkyl bedeutet, $R^4$ H, OH, $C_{1-4}$-Alkyl, O-$C_{1-4}$-Alkyl, O-Benzyl, $CF_3$, O-$CF_3$, Cl, F oder $OR^8$ bedeutet, $R^5$ H, OH, $C_{1-4}$-Alkyl, O-$C_{1-4}$-Alkyl, O-Benzyl, $CHF_2$, $CF_3$, O-$CF_3$, Cl, F oder $OR^8$ darstellt und $R^6$ H, OH, $C_{1-4}$-Alkyl, O-$C_{1-4}$-Alkyl, O-Benzyl, $CF_3$, O-$CF_3$, Cl, F oder $OR^8$ bedeutet, mit der Maßgabe, daß zwei der Reste $R^4$, $R^5$ oder $R^6$ H sind, oder $R^4$ und $R^5$ zusammen $-CH=C(R^9)-O-$ oder $-CH=C(R^9)-S-$ bedeuten mit der Maßgabe, daß $R^6$ H ist, oder $R^5$ und $R^6$ zusammen $-CH=CH-C(OR^{10})=CH-$ bedeuten mit der Maßgabe, daß $R^4$ H ist, $R^7$ $C_{1-8}$-Alkyl, $C_{3-8}$-Cycloalkyl, O-$C_{1-4}$-Alkyl, O-Benzyl, $CF_3$, Cl oder F bedeutet, $R^8$ CO-$C_{1-5}$-Alkyl, PO(O-$C_{1-4}$-Alkyl)$_2$, CO-$C_6H_4$-$R^{11}$, CO(O-$C_{1-5}$-Alkyl), CO-$CHR^{12}$-$NHR^{13}$, CO-NH-$C_6H_3$-$(R^{14})2$ oder eine unsubstituierte oder substituierte Pyridyl-, Thienyl-, Thiazoyl- oder Phenylgruppe bedeutet, $R^9$ H oder $C_{1-4}$-Alkyl bedeutet, $R^{10}$ H oder $C_{1-3}$-Alkyl bedeutet, $R^{11}$ OC(O)-$C_{1-3}$-Alkyl in ortho-Stellung oder $CH_2$-N-$(R^{15})_2$ in meta- oder para-Stellung, wobei $R^{15}$ $C_{1-4}$-Alkyl oder beide Reste $R^{15}$ zusammen mit N den 4-Morpolino-Rest bilden, bedeutet, $R^{12}$ und $R^{13}$ gleich oder verschieden sind und H, $C_{1-6}$-Alkyl oder $C_{3-8}$-Cycloalkyl oder $R^{12}$ und $R^{13}$ zusammen $-(CH_2)_{3-8}-$ bedeuten, $R^{14}$ H, OH, $C_{1-7}$-Alkyl, O-$C_{1-7}$-Alkyl, Phenyl, O-Aryl, $CF_3$, Cl oder F bedeutet, mit der Maßgabe, daß die beiden Reste $R^{14}$ gleich oder verschieden sind, in Form ihrer Basen und/oder Salze von physiologisch verträglichen Säuren, als Enantiomere oder Racemate mit der Maßgabe, daß das Racemat der Verbindung der Formel I, in der $R^1$ und $R^2$ zusammen $-(CH_2)_3-$ sind, $R^3$, $R^4$ und $R^6$ H bedeuten und $R^5$ $OCH_3$ ist, ausgenommen ist.

Bevorzugte Dimethyl-(3-aryl-3-but-3-enyl)-aminverbindungen entsprechen der Formel I mit $R^1$ und $R^2$ zusammen $-(CH_2)_{2-4}-$ oder $-(CH_2)_2-CHR^7$, $R^3$ H oder $C_{1-3}$-Alkyl, $R^4$ H, OH, $CF_3$, Cl, F oder $OR^8$, $R^5$ H,

OH, $C_{1-4}$-Alkyl, O-$C_{1-4}$-Alkyl, O-Benzyl, $CHF_2$, $CF_3$, Cl, F oder $OR^8$ und $R^6$ H, OH, O-$C_{1-4}$-Alkyl, O-Benzyl, $CF_3$, Cl, F oder $OR^8$, mit der Maßgabe, daß zwei der Reste $R^4$, $R^5$ oder $R^6$ H sind, oder $R^4$ und $R^5$ zusammen -CH=C($R^9$)-O- oder -CH=C($R^9$)-S-, mit der Maßgabe, daß $R^6$ H ist, oder $R^5$ und $R^6$ zusammen -CH=CH-C($OR^{10}$)=CH-, mit der Maßgabe, daß $R^4$ H ist, und $R^7$ $C_{1-4}$-Alkyl, $CF_3$, Cl oder F. Besonders eignen sich Dimethyl-(3-aryl-but-3-enyl)-aminverbindungen der Formel I, in der $R^1$ und $R^2$ zusammen - $(CH_2)_{2-3}$- oder -$(CH_2)_2$-$CHR^7$ bedeuten, $R^3$ H, $CH_3$ oder $CH_2CH_3$ bedeutet, $R^4$ H oder OH, $R^5$ H, OH, $OCH_3$, $CHF_2$ oder $OR^8$ und $R^6$ H, OH oder $CF_3$ bedeuten, mit der Maßgabe, daß zwei der Reste $R^4$, $R^5$ oder $R^6$ H sind, oder $R^4$ und $R^5$ zusammen -CH=C($CH_3$)-S- darstellen, mit der Maßgabe, daß $R^6$ H ist, oder $R^5$ und $R^6$ zusammen -CH=CH-C(OH)=CH-bedeuten, mit der Maßgabe, daß $R^4$ H ist, und $R^8$ CO-$C_6H_4$-$R^{11}$ mit $R^{11}$ OC(O)-$C_{1-3}$-Alkyl in ortho-Stellung darstellt. Insbesondere bevorzugt werden Dimethyl-(3-aryl-but-3-enyl)-aminverbindungen mit $R^1$ und $R^2$ zusammen -$(CH_2)_{2-3}$- oder -$(CH_2)_2$-CH($CH_3$)-, $R^3$ H oder $CH_3$, $R^4$ H, $R^5$ OH oder $OR^8$, $R^6$ H und $R^8$ CO-$C_6H_4$-$R^{11}$ mit $R^{11}$ OC(O)$CH_3$ in ortho-Stellung.

Weiterer Erfindungsgegenstand ist ein Verfahren zur Herstellung von Dimethyl-(3-aryl-but-3-enyl)-aminverbindungen der Formel I, in der $R^1$ und $R^2$ zusammen -$(CH_2)_{2-4}$-, -$(CH_2)_2$-$CHR^7$ oder -$CH_2$-$CHR^7$-$CH_2$- darstellen, $R^3$ H oder $C_{1-5}$-Alkyl bedeutet, $R^4$ H, $C_{1-4}$-Alkyl, O-$C_{1-4}$-Alkyl, O-Benzyl, $CF_3$, O-$CF_3$, Cl oder F bedeutet, $R^5$ H, $C_{1-4}$-Alkyl, O-$C_{1-4}$-Alkyl, O-Benzyl, $CHF_2$, $CF_3$, O-$CF_3$, Cl oder F darstellt und $R^6$ H, $C_{1-4}$-Alkyl, O-$C_{1-4}$-Alkyl, O-Benzyl, $CF_3$, O-$CF_3$, Cl oder F bedeutet, mit der Maßgabe, daß zwei der Reste $R^4$, $R^5$ oder $R^6$ H sind, oder $R^4$ und $R^5$ zusammen -CH=C($R^9$)-O- oder -CH=C($R^9$)-S- bedeuten mit der Maßgabe, daß $R^6$ H ist, oder $R^5$ und $R^6$ zusammen -CH=CH-C($OR^{10}$)=CH-bedeuten, mit der Maßgabe, daß $R^4$ H ist, $R^7$ $C_{1-8}$-Alkyl, $C_{3-8}$-Cycloalkyl, O-$C_{1-4}$-Alkyl, O-Benzyl, $CF_3$, Cl oder F bedeutet, $R^9$ H oder $C_{1-4}$-Alkyl und $R^{10}$ H oder $C_{1-3}$-Alkyl bedeuten, wobei die Verbindung der Formel I, in der $R^1$ und $R^2$ zusammen -$(CH_2)_3$- bedeuten, $R^3$, $R^4$ und $R^6$ H sind und $R^5$ $OCH_3$ ist, ausgenommen ist, welches dadurch gekennzeichnet ist, daß man ein β-Dimethylaminoketon der Formel II

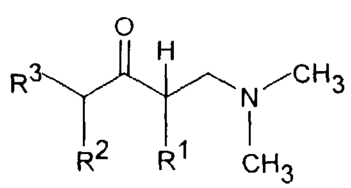

mit einer metallorganischen Verbindung der Formel III

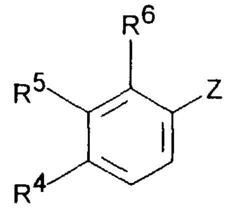

in der Z MgCl, MgBr, MgI oder Li bedeutet, zu einem tertiären Alkohol der Formel IV

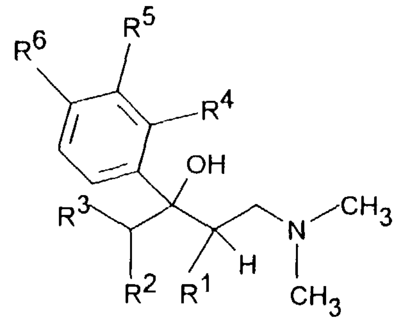

umsetzt, welcher anschließend zu einer Verbindung der Formel I dehydratisiert wird.

Die Reaktion eines β-Dimethylaminketons mit einer Grignard-Verbindung der Formel III, in der Z MgCl, MgBr oder MgJ bedeutet, oder mit einer lithiumorganischen Verbindung der Formel III kann in einem aliphatischen Ether, beispielsweise Diethylether und/oder Tetrahydrofuran, bei Temperaturen zwischen -70° und +60° C durchgeführt werden. Die Umsetzung mit einer Grignard-Verbindung kann mit oder ohne Zusatz eines Mitführreagenzes, vorzugsweise 1,2-Dibromethan, erfolgen. Lithiumorganische Verbindungen der Formel III lassen sich durch Umsetzung einer Verbindung der Formel III, in der Z Cl, Br oder J bedeutet, mit beispielsweise einer n-Butyllithium/Hexan-Lösung durch Halogen/Lithiumaustausch erhalten.

Die erhaltenen tertiären Alkohole der Formel IV lassen sich mit Säuren, insbesondere Ameisensäure oder Salzsäure, bei Temperaturen zwischen 0° und 100° C dehydratisieren.

Weiterer Erfindungsgegenstand ist ein Verfahren zur Herstellung einer Dimethyl-(3-aryl-but-3-enyl)-aminverbindung der Formel I, in der $R^1$ und $R^2$ zusammen -$(CH_2)_{2-4}$-, -$(CH_2)_2$-$CHR^7$ oder -$CH_2$-$CHR^7$-$CH_2$- bedeuten, $R^3$ H oder $C_{1-5}$-Alkyl bedeutet, einer der Reste $R^4$, $R^5$ oder $R^6$ OH bedeutet und die anderen beiden Reste H sind, $R^7$ $C_{1-8}$-Alkyl, $C_{3-8}$-Cycloalkyl, O-$C_{1-4}$-Alkyl, O-Benzyl, $CF_3$, Cl oder F bedeutet, welches dadurch gekennzeichnet ist, daß man eine Verbindung der Formel I, in der einer der Reste $R^4$, $R^5$ oder $R^6$ O-$CH_3$ bedeutet und die anderen beiden Reste H sind, mit Diisobutylaluminiumhydrid umsetzt oder eine Verbindung der Formel I, in der einer der Reste $R^4$, $R^5$ oder $R^6$ O-Benzyl bedeutet und die anderen beiden Reste H sind, reduktiv debenzyliert.

Die Umsetzung einer Dimethyl-(3-aryl-but-3-enyl)-aminverbindung mit Diisobutylaluminiumhydrid wird üblicherweise in einem aromatischen Kohlenwasserstoff, beispielsweise Toluol, bei einer Temperatur zwischen 60° und 130° C durchgeführt (Synthesis 1975, 617; DE 24 09 990, DE 24 09 991; Chem. Abstr. 84 59862 (1974)).
Die reduktive Debenzylierung einer erfindungsgemäßen Verbindung der Formel I, in der einer der Reste $R^4$, $R^5$ oder $R^6$ O-Benzyl bedeutet, läßt sich in Gegenwart von Platin oder Paladium auf einem Trägermaterial, beispielsweise Aktivkohle, in Gegenwart von Wasserstoff in einem Lösungsmittel, beispielsweise Essigsäure oder $C_{1-4}$-Alkylalkohol, bei Drücken zwischen 1 und 100 bar und Temperaturen zwischen 20° und 100° C durchführen.

Dimethyl-(3-aryl-but-3-enyl)-aminverbindungen der allgemeinen Formel I, in der einer oder mehrere der Aromatensubstituenten $R^4$, $R^5$ und $R^6$ $OR^8$ bedeuten und $OR^8$ eine Phosphat-, Carbonat-, Carbamat-, Carboxylat- oder Aryloxy- oder Heteroaryloxygruppe darstellt, lassen sich durch Umsetzung einer entsprechenden Dimethyl-[3-(hydroxy-phenyl)-but-3-enyl]-aminverbindung der Formel I, in der $R^4$, $R^5$ und/oder $R^6$ eine OH-Gruppe bedeuten, in Form eines Alkalisalzes mit einem Dialkylchlorophosphat, mit einem Alkylchloroformiat, mit einem Aryl- oder Heteroarylisocyanat, mit einem Carbonsäurechlorid oder einem Aryl- oder Heteroarylhalogenid erhalten. Diese Umsetzungen werden üblicherweise in einem Lösungsmittel, beispielsweise Toluol, Dichlormethan, Diethylether und/oder Tetrahydrofuran bei Temperaturen zwischen -15° und +110° C durchgeführt (Drugs of the Future 16, 443 (1991); J. Med. Chem 30, 2008 (1989) und 32, 2503 (1989); J. Org. Chem. 43, 4797 (1978); Tetrahedron Lett. 1977, 1571; J. Pharm. Sci. 57, 774 (1968)). Die Umsetzungen mit einem Aryl- oder Heteroarylhalogenid werden unter Zusatz von Kupferpulver und/ oder einem Kupfer-I-halogenid als Katalysator durchgeführt.

Dimethyl-(3-aryl-but-3-enyl)-aminverbindungen der Formel I, in der $OR^8$ eine $\alpha$-Aminocarboxylatgruppe darstellt, sind durch Umsetzung einer entsprechenden Dimethyl-[3-(hydroxy-phenyl)-but-3-enyl]-aminverbindung der Formel I, in der $R^4$, $R^5$ und/oder $R^6$ eine OH-Gruppe bedeuten, mit einer entsprechenden 2-t-Butoxycarbonylamino-carbonsäure unter Verwendung von Triethylamin und Kupplungsreagenzien, wie Benzotriazol-1-yl-oxy-tripyrrolidinophosphoniumhexafluorophosphat in einem Lösungsmittel, beispielsweise Dichlormethan, erhältlich.

Die Verbindungen der Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in an sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylohlorsilan in wäßriger Lösung.

Die erfindungsgemäßen Verbindungen haben eine ausgeprägte analgetische Wirkung und sind toxikologisch unbedenklich. Sie eignen sich daher als pharmazeutische Wirkstoffe. Dementsprechend ist Erfindungsgegenstand auch die Verwendung einer Dimethyl-(3-aryl-but-3-enyl)-aminverbindung der Formel I als Wirkstoff in Arzneimitteln, vorzugsweise als Wirkstoff in Schmerzmitteln.

Erfindungsgemäße Arzneimittel enthalten neben mindestens einer Dimethyl-(3-aryl-but-3-enyl)-aminverbindung der Formel I Trägermaterialien, Füllstoffe, Lösungsmittel, Verdünnungsmittel, Farbstoffe und/oder Bindemittel. Die Auswahl der Hilfsstoffe sowie die einzusetzen-den Mengen hängen davon ab, ob das Arzneimittel oral, intravenös, intraperitoneal, intradermal, intramuskulär, intranasal, buccal oder örtlich, zum Beispiel auf Infektionen an der Haut, an den Schleimhäuten oder an den Augen, appliziert werden soll. Für die orale Applikation eignen sich Zubereitungen in Form von Tabletten, Dragees, Kapseln, Granulaten Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot in gelöster Form oder in einem Pflaster, gegebenenfalls unter Zusatz von die Hauptpenetration fördernden Mitteln, sind Beispiele für geeignete perkutane Zubereitungsformen. Aus oral oder perkutan anwendbaren Zubereitungsformen können die erfindungsgemäßen Verbindungen verzögert freigesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Ublicherweise werden 10 bis 500 mg pro kg wenigstens einer Dimethyl-(3-aryl-but-3-enyl)-aminverbindung der Formel I appliziert.

**Beispiele**

**Herstellung erfindungsgemäßer Verbindungen**

Die Angabe Ether bedeutet Diethylether.

Als stationäre Phase für die Säulenchromatographie wurde Kieselgel 60 (0,040 - 0,063 mmm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Racemattrennungen wurden auf einer Chiracel OD Säule der Firma Daicel Chemical Industries, LTD

durchgeführt.

Die Mischungsverhältnisse der Laufmittel für alle chromatographischen Untersuchungen sind in Volumen/Volumen angegeben.

**Beispiel 1**

(RS)-Dimethyl-[2-(4-trifluormethyl-phenyl)-cyclopent-2-enylmethyl]-amin, Hydrochlorid (27)

(RS)-2-Dimethylaminomethyl-cyclopentanon und 1 -Brom-4-trifluormethyl-benzol wurden unter den in Beispiel 1 (1. Stufe) angegebenen Bedingungen umgesetzt. 30 g des erhaltenen Rohproduktes wurden auf eine Säule, gefüllt mit Kieselgel, gegeben. Die Elution mit Essigsäureethylester/Methanol = 5: 1 ergab 11,6 g Base, die mit Trimethylchlorsilan/Wasser in 2-Butanon in 12,0 g (21 % der Theorie) (1RS,2RS)-2-Dimethylaminomethyl-1-(4-trifluormethyl-phenyl)-cyclopentanol, Hydrochlorid (28) mit einem Schmelzpunkt von 213 - 214° C überführt wurden. 32,4 g (0,1 mol) des Hydrochlorids (28) wurden gemäß Beispiel 5 mit 450 ml konzentrierter Ameisensäure umgesetzt. Die erhaltene Rohbase wurde auf eine Säule, gefüllt mit Kieselgel, gegeben Die Elution mit Diisopropylether/Methanol = 7 : 1 ergab 9,6 g Base, die mit Trimethylchlorsilan/Wasser in 2-Butanon in 8,9 g (29 % der Theorie Hydrochlorid (27) mit einem Schmelzpunkt von 219-220° C überführt wurden.

**Beispiel 2**

Enantiomere von (27):

(+)-(S)-Dimethyl-[2-(4-trifluormethyl-phenyl)-cyclopent-2-enylmethyl]-amin, Hydrochlorid (+27)
und

(-)-(R)-Dimethyl-[2-(4-trifluormethyl-phenyl)-cyclopent-2-enylmethyl]-amin, Hydrochlorid (-27)

Aus (27) wurde mit Dichlormethan/Natronlauge die Base freigesetzt. Nach dem Trocknen der Lösung wurde Dichlormethan im Vakuum abdestilliert. Das Racemat wurde dann auf einer chiralen HPLC-Säule getrennt. Aus den erhaltenen Enantiomeren wurden durch Umsetzung mit konzentrierter Salzsäure in Aceton die Hydrochloride mit einem Schmelzpunkt von 244 - 246° C hergestellt.

(+27): Ausbeute: 42 % der Theorie
$[\alpha]_D^{RT}$ = +33,8° (c = 1,00; Methanol)

(-27): Ausbeute: 44 % der Theorie
$[\alpha]_D^{RT}$ = -34,3° (c = 1,06; Methanol)

**Beispiel 3**

(RS)-2-(6-Dimethylaminomethyl-cyclohex-1 -enyl)-phenol, Hydrochlorid (29)

Ausgehend von (RS)-2-Dimethylaminomethyl-cyclohexanon und 1 -Brom-2-methoxy-benzol wurde unter den in Beispiel 1 (1. Stufe) angegebenen Bedingungen unter Verwendung von Ether als Lösungsmittel (1RS,2RS)-2-Dimethylaminomethyl-1-(2-methoxy-phenyl)-cyclohexanol, Hydrochlorid (30) in einer Ausbeute von 47 % erhalten. Aus (30) wurde mit Dichlormethan/Natronlauge die Base freigesetzt. Nach dem Trocknen der Lösung wurde Dichlormethan im Vakuum abdestilliert. 30,0 g (0,1 mol) der Base wurden gemäß Beispiel 2 mit Diisobutylaluminiumhydrid umgesetzt. Es wurden 22,7 g (78 % der Theorie) (1RS,2RS)-2-(2-Dimethylaminomethyl-1-hydroxy-cyclo-hexyl)-phenol, Hydrochlorid (31) mit einem Schmelzpunkt von 168 - 170° C erhalten. 28,6 g (0,1 mol) der Verbindung (31) wurden gemäß Beispiel 5 mit 450 ml konzentrierter Ameisensäure umgesetzt. Die erhaltene Rohbase wurde auf eine Säule, gefüllt mit Kieselgel, gegeben und mit Diisopropylether/Methanol = 7 : 1 eluiert. Es wurden 21 g Base gewonnen, aus der mit konzentrierter Salzsäure in Aceton 18,6 g (69 % der Theorie) Hydrochlorid (29) mit einem Schmelzpunkt von 168° C erhalten wurde.

**Beispiel 4**

Enantiomere von (29):

(-)-(R)-2-(6-Dimethylaminomethyl-cyclohex-1-enyl)-phenol, Hydrochlorid (-29)
und

(+)-(S)-2-(6-Dimethylaminomethyl-cyclohex-1-enyl)-phenol, Hydrochlorid (+29)

Aus (29) wurde mit Dichlormethan/wäßriger Natriumhydrogencarbonatlösung die Base freigesetzt. Nach dem Trocknen der Lösung wurde Dichlormethan im Vakuum abdestilliert. Das Racemat wurde dann auf einer chiralen HPLC-Säule getrennt. Aus den erhaltenen Enantiomeren wurden durch Umsetzung mit konzentrierter Salzsäure in Aceton die Hydrochloride mit einem Schmelzpunkt von 271 - 272° C isoliert.

(+29): Ausbeute: 43 % der Theorie
$[\alpha]_D^{RT}$ = +24,1° (c = 0,96; Methanol)

(-29): Ausbeute: 44 % der Theorie
$[\alpha]_D^{RT}$ = -23,5° (c = 0,94; Methanol)

**Beispiel 5**

(RS)-Dimethyl-[2-(4-trifluoromethyl-phenyl)-cyclohex-2-enylmethyl]-amin, Hydrochlorid (32)

(RS)-2-Dimethylaminomethyl-cyclohexanon und 1-Brom-4-trifluormethyl-benzol wurden unter den in Beispiel 1 (1. Stufe) angegebenen Bedingungen umgesetzt. 30 g des Rohproduktes wurden auf eine Säule, gefüllt mit Kieselgel, gegeben. Die Elution mit Essigsäureethylester/Methanol = 5 : 1 ergab 18,9 g Base, die mit Trimethylchlorsilan/Wasser in 2-Butanon in 16,4 g (37% der Theorie) (1RS,2RS)-2-Dimethylaminomethyl-1-(4-trifluormethyl-phenyl)-cyclohexanol, Hydrochlorid (33) mit einem Schmelzpunkt von 234° C überführt wurden. 33,7 g (0,1 mol) des Hydrochlorids (33) wurden gemäß Beispiel 5 mit 450 ml konzentrierter Ameisensäure umgesetzt. Die erhaltene Rohbase wurde auf eine Säule, gefüllt mit kieselgel, gegeben und mit Diisopropylether/ Methanol = 7 : 1 eluiert. Es wurden 12,3 g Base erhalten, die mit Trimethylchlorsilan/Wasser in 2-Butanon in 10,4 g (32,5 % der Theorie Hydrochlorid (32) mit einem Schmelzpunkt von 205 - 206 °C überführt wurden.

**Beispiel 6**

(RS)-Dimethyl-[2-(2-methyl-benzo[b]thiophen-4-yl)-cyclohex-2-enylmethyl]-amin, Hydrochlorid (34)

(RS)-2-Dimethylaminomethyl-cyclohexanon und 4-Brom-2-methyl-benzo[b]thiophen wurden unter den in Beispiel 1 (1. Stufe) angegebenen Bedingungen unter Verwendung von Ether als Lösungsmittel und 1,2-Dibromethan als Mitführreagenz umgesetzt. 25 g des Rohproduktes wurden auf eine Säule, gefüllt mit Kieselgel, gegeben. Die Elution mit Essigsäureethylester/Methanol = 1 : 1 ergab 12,6 g Base, die mit Trimethylchlorsilan/Wasser in 2-Butanon in 10,4 g (29 % der Theorie) (1RS,2RS)-2-Dimethylaminomethyl-1-(2-methyl-benzo[bjthiophen-4-yl)-cyclohexanol, Hydrochlorid (35) mit einem Schmelzpunkt von 204° C überführt wurden. 34,0 g (0,1 mol) des Hydrochlorids (35) wurden gemäß Beispiel 5 mit 450 ml konzentrierter Ameisensäure umgesetzt. Die auf diese Weise erhaltene Rohbase (28,4 g) wurde auf eine Säule, gefullt mit Kieselgel, gegeben. Die Elution mit Ether ergab 17,5 g Base, die mit Trimethylchlorsilan/Wasser in 2-Butanon in 15,2 g (54,8 % der Theorie) Hydrochlorid (34) mit einem Schmelzpunkt von 179 - 182° C überfuhrt wurden.

**Beispiel 7**

(-)-(3S,6R)-3-(6-Dimethylaminomethyl-3-methyl-cyclohex-1-enyl)-phenol, Hydrochlorid (-36)
und
(+)-(3R,6S)-3-(6-Dimethylaminomethyl-3-methyl-cyclohex-1-enyl)-phenol, Hydrochlorid (+36)

1. Stufe:

(1RS,2RS,5SR)-2-Dimethylaminomethyl-1-(3-methoxy-phenyl)-5-methyl-cyclohexanol, Hydrochlorid (37)

95 ml (750 mmol) 1-Brom-3-methoxy-benzol wurden in 425 ml trockenem Tetrahydrofuran gelöst und auf -75° C gekühlt. Nach Zugabe von 750 mmol 1,6 molarer n-Butyllithiumlösung in Hexan wurde eine Stunde bei -75° C gerührt. Anschließend wurden 82 g (484 mmol) (2RS,5SR)-2-Dimethylaminomethyl-5-methyl-cyclohexanon, hergestellt aus 3-Methylcyclohexanon, Dimethylaminhydrochlorid und Paraformaldehyd in Eisessig, gelöst in 120 ml trockenem Tetrahydrofuran, zugetropft. Innerhalb von 2,5 Stunden wurde das Reaktionsgemisch auf Raumtemperatur erwärmt.

Zur Aufarbeitung wurden unter Eisbadkühlung 200 ml Wasser zugetropft, so daß die Innentemperatur 15° C nicht überstieg. Nach Phasentrennung wurde die wäßrige Phase dreimal mit 50 ml Essigsäureethylester extrahiert. Die vereinigten organischen Phasen wurden über Natriumsulfat getrocknet. Nach destillativer Entfernung des Lösungsmittels wurde der Rückstand in 700 ml Aceton gelöst und mit Trimethylchlorsilan/Wasser versetzt. Bei 4 - 5° C kristallisierten 67 g (48 % der Theorie) Hydrochlorid (37) mit einem Schmelzpunkt von 173 - 175° C aus.

2. Stufe:

Enantiomere von (37):

(+)-(1R,2R,5S)-2-Dimethylaminomethyl-1-(3-methoxy-phenyl)-5-methyl-cyclohexanol, Hydrochlorid (+37)
und

(-)-(1S,2S,5R)-2-Dimethylaminomethyl-1-(3-methoxy-phenyl)-5-methyl-cyclohexanol, Hydrochlorid (-37)

Aus (37) wurde mit Dichlormethan/Natronlauge die Base freigesetzt. Nach dem Trocknen der Lösung wurde Dichlormethan im Vakuum abdestilliert. Das Racemat wurde dann auf der chiralen HPLC-Säule getrennt. Aus den erhaltenen Enantiomeren wurden durch Umsetzung mit Trimethyl-. chlorsilan/Wasser in 2-Butanon die Hydrochloride mit einem Schmelzpunkt von 151 - 153° C isoliert.

(+37): Ausbeute: 43 % der Theorie
$[\alpha]_D^{RT}$ = +36,4° (c = 1,01; Methanol)

(-37): Ausbeute: 44 % der Theorie
$[\alpha]_D^{RT}$ = -37,7° (c = 1,01; Methanol)

3. Stufe:

(-)-(1R,4S)-[2-(3-Methoxy-phenyl)-4-methyl-cyclohex-2-enylmethyl]-dimethylamin, Hydrochlorid (-38) und

(+)-(1S,4R)-[2-(3-Methoxy-phenyl)-4-methyl-cyclohex-2-enylmethyl]-dimethylamin, Hydrochlorid (+38)

Die Methoxyverbindungen (-37) und (+37) aus der 2. Stufe wurden unter den in Beispiel 5 angegebenen Bedingungen in die Hydrochloride (+38) und (-38) in einer Ausbeute von 87 % der Theorie und mit einem Schmelzpunkt von 122 - 123° C überführt.

4. Stufe:

(-)-(3S,6R)-3-(6-Dimethylaminomethyl-3-methyl-cyclohex-1-enyl)-phenol, Hydrochlorid (-36) und

(+)-(3R,6S)-3-(6-Dimethylaminomethyl-3-methyl-cyclohex-1-enyl)-phenol, Hydrochlorid (+36)

Aus den nach Stufe 3 erhaltenen Basen wurden unter den in Beispiel 2 angegebenen Bedingungen durch Umsetzung mit Diisobutylaluminiumhydrid und anschließender Hydrochloridfällung mit Trimethylchlorsilan/Wasser in 2-Butanon die Hydrochloride (-36) und (+36) in einer Ausbeute von 79 % der Theorie und einem Schmelzpunkt von 131 - 133° C erhalten.

(-36): $[\alpha]_D^{RT}$ = - 75,5° (c = 0,96; Methanol)

(+36): $[\alpha]_D^{RT}$ = + 77,7° (c = 1,08; Methanol)

**Beispiel 8**

(-)-(R)-3-(6-Dimethylaminomethyl-cyclohex-1-enyl)-phenol, Hydrochlorid (-39)

28,8 g (0,1 mol) (+)-(1R,2R)-3-(2-Dimethylaminomethyl-1-hydroxycyclohexyl)-phenol, Hydrochlorid wurden in 450 ml konzentrierter Ameisensäure gelöst und zwei Stunden unter Rückfluß erhitzt. Anschließend wurde die Ameisensäure im Wasserstrahlvakuum abdestilliert und aus dem Rückstand mit Dichlormethan/wäßriger Natriumcarbonatlösung die Base freigesetzt, aus der mit konzentrierter Salzsäure in Aceton 21,8 g (81,4 % der Theorie) Hydrochlorid (-39) mit einem Schmelzpunkt von 216-217° C erhalten wurden.

(-39): $[\alpha]_D^{RT}$ = -96,6° (c = 1,04; Methanol)

**Beispiel 9**

(+)-(S)-3-(6-Dimethylaminomethyl-cyclohex-1-enyl)-phenol, Hydrochlorid (+39)

Unter den in Beispiel 26 angegebenen Bedingungen wurden aus 28,8 g (0,1 mol) (-)-(1S,2S)-3-(2-Dimethylaminomethyl-1-hydroxy-cyclohexyl)-phenol, Hydrochlorid 21,8 g (81,4 % der Theorie) Hydrochlorid (+39) mit einem Schmelzpunkt von 216 - 217° C erhalten.

(+39): $[\alpha]_D^{RT}$ = +89,0° (c = 0,99; Methanol)

**Pharmakologische Untersuchungen**

**Analgesieprüfung im Writhing-Test an der Maus**

Die analgetische Wirksamkeit wurde im Phenylchinon-induzierten Writhing an der Maus, modifiziert nach I.C. Hendershot, J. Forsaith, J. Pharmacol. Exp. Ther. 125, 237-240 (1959) untersucht. Dazu wurden männliche NMRI-Mäuse mit einem Gewicht von 25 - 30 g eingesetzt. Gruppen von 10 Tieren pro Substanzdosis wurden 10 Minuten nach intravenöser Gabe einer erfindungsgemäßen Verbindung 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45° C) intraperitoneal appliziert. Die Tiere wurden einzeln in Beobachtungskäfige gesetzt. Mittels eines Drucktastenzählers wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Aus der dosisabhängigen Abnahme der Writhingreaktionen im Vergleich zu parallel untersuchten Tiergruppen, denen keine erfindungsgemäße Verbindungen appliziert wurde, wurden mittels Regressionsanalyse (Auswerteprogramm Martens EDV Service, Eckental) die $ED_{50}$-Werte der Writhingreaktion berechnet.

Alle untersuchten erfindungsgemäßen Verbindungen zeigten eine ausgeprägte analgetische Wirkung, die im Vergleich zu Tramadol verstärkt war.

Die Ergebnisse sind in der nachfolgenden Tabelle zusammengefaßt.

Tabelle

| Analgesieprüfung im Writhing-Test an der Maus | |
|---|---|
| erfindungsgemäße Verbindung hergestellt nach Beispiel | $ED_{50}$ (mg/kg) |
| 2 (+)-Enantiomer | 1,40 |
| 4 (-)-Enantiomer | 2,12 |
| 6 | 1,35 |
| 7 (-)-Enantiomer | 0,90 |
| 8 (-)-Enantiomer | 1,04 |
| 9 (+)-Enantiomer | 1,60 |
| zum Vergleich: Tramadol | 3,68 |

**Patentansprüche**

**1.** Dimethyl-(3-aryl-but-3-enyl)-aminverbindungen der Formel I,

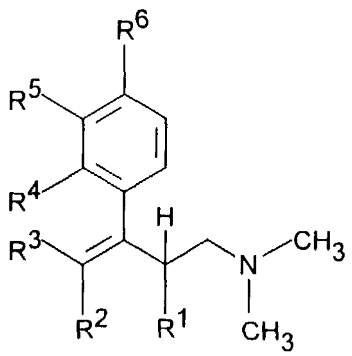

in der $R^1$ und $R^2$ zusammen $-(CH_2)_{2-4}-$, $-(CH_2)_2-CHR^7$ oder $-CH_2-CHR^7-CH_2-$ darstellen,

$R^3$ H oder $C_{1-5}$-Alkyl bedeutet,

$R^4$ H bedeutet,

$R^5$ OH, $C_{1-4}$-Alkyl, O-$C_{1-4}$-Alkyl, O-Benzyl, $CHF_2$, $CF_3$, O-$CF_3$, Cl, F oder $OR^8$ darstellt und

$R^6$ H bedeutet,

$R^7$ $C_{1-8}$-Alkyl, $C_{3-8}$-Cycloalkyl, O-$C_{1-4}$-Alkyl, O-Benzyl, $CF_3$, Cl oder F bedeutet

$R^8$ CO-$C_{1-5}$-Alkyl, PO(O-$C_{1-4}$-Alkyl)$_2$, CO-$C_6H_4$-$R^{11}$, CO(O-$C_{1-5}$-Alkyl), CO-$CHR^{12}$-$NHR^{13}$, CO-NH-$C_6H_3$-$(R^{14})_2$ oder eine unsubstituierte oder substituierte Pyridyl-, Thienyl-, Thiazoyl- oder Phenylgruppe bedeutet,

$R^{11}$ OC(O)-$C_{1-3}$-Alkyl in ortho-Stellung oder $CH_2$-N-$(R^{15})_2$ in meta- oder para-Stellung, wobei $R^{15}$ $C_{1-4}$-Alkyl oder beide Reste $R^{15}$ zusammen mit N den 4-Morpolino-Rest bilden, bedeutet

$R^{12}$ und $R^{13}$ gleich oder verschieden sind und H, $C_{1-6}$-Alkyl oder $C_{3-8}$-Cycloalkyl oder $R^{12}$ und $R^{13}$ zusammen $-(CH_2)_{3-8}-$ bedeuten,

$R^{14}$ H, OH, $C_{1-7}$Alkyl, O-$C_{1-7}$-Alkyl, Phenyl, O-Aryl, $CF_3$, Cl oder F bedeutet, mit der Maßgabe, daß die beiden Reste $R^{14}$ gleich oder verschieden sind,

in Form ihrer Basen und/oder Salze von physiologisch verträglichen Säuren, als Enantiomere oder Racemate mit der Maßgabe, daß die Racemate der Verbindungen der Formel I,

in der $R^1$ und $R^2$ zusammen $-(CH_2)_3-$ sind, $R^3$, $R^4$ und $R^6$ H bedeuten und $R^5$ $OCH_3$ ist, oder

in der $R^1$ und $R^2$ zusammen $-(CH_2)_3-$ sind, $R^3$, $R^4$ und $R^6$ H bedeuten und $R^5$ O-CO-$CH_3$ ist, oder

in der $R^1$ und $R^2$ zusammen $-(CH_2)_2-$ sind, $R^3$, $R^4$ und $R^6$ H bedeuten und $R^5$ OH ist, als Hydrochlorid, oder

in der $R^1$ und $R^2$ zusammen $-(CH_2)_3-$ sind, $R^3$, $R^4$ und $R^6$ H bedeuten und $R^5$ OH ist, als Hydrochlorid, oder

in der $R^1$ und $R^2$ zusammen $-(CH_2)_4-$ sind, $R^3$, $R^4$ und $R^6$ H bedeuten und $R^5$ OH ist, als Hydrochlorid,

ausgenommen sind.

**2.** Dimethyl-(3-aryl-but-3-enyl)-aminverbindungen gemäß Anspruch 1, dadurch gekennzeichnet, daß

$R^1$ und $R^2$ zusammen $-(CH_2)_{2-4}-$ oder $-(CH_2)_2-CHR^7$ bedeuten,

$R^3$ H oder $C_{1-3}$-Alkyl bedeutet,

$R^4$ H bedeutet,

$R^5$ OH, $C_{1-4}$-Alkyl, O-$C_{1-4}$-Alkyl, O-Benzyl, $CHF_2$, $CF_3$, Cl, F oder $OR^8$ darstellt und

$R^6$ H bedeutet,

$R^7$ $C_{1-4}$-Alkyl, $CF_3$, Cl oder F bedeutet.

**3.** Dimethyl-(3-aryl-but-3-enyl)-aminverbindungen gemäß Anspruch 1 oder 2, dadurch gekennzeichnet, daß

$R^1$ und $R^2$ zusammen $-(CH_2)_{2-3}$- oder $-(CH_2)_2$-$CHR^7$ bedeuten,

$R^3$ H, $CH_3$ oder $CH_2CH_3$ bedeutet,

$R^4$ H, $R^5$ OH, $OCH_3$, $CHF_2$ oder $OR^8$ und $R^6$ H bedeutet,

$R^8$ CO-$C_6H_4$-$R^{11}$ mit $R^{11}$ OC(O)-$C_{1-3}$-Alkyl in ortho-Stellung darstellt.

**4.** Dimethyl-(3-aryl-but-3-enyl)-aminverbindungen gemäß einem oder mehreren der Ansprüche 1 bis 3, dadurch gekennzeichnet, daß

$R^1$ und $R^2$ zusammen $-(CH_2)_{2-3}$- oder $-(CH_2)_2$-$CH(CH_3)$- darstellen,

$R^3$ H oder $CH_3$ bedeutet,

$R^4$ H ist, $R^5$ OH oder $OR^8$ bedeutet, $R^6$ H ist, und $R^8$ CO-$C_6H_4$-$R^{11}$ mit $R^{11}$ OC(O)-$CH_3$ in ortho-Stellung bedeutet.

**5.** Verfahren zur Herstellung einer Dimethyl-(3-aryl-but-3-enyl)-aminverbindung der Formel I,

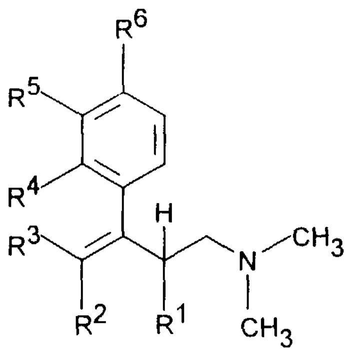

in der $R^1$ und $R^2$ zusammen $-(CH_2)_{2-4}$-, $-(CH_2)_2$-$CHR^7$ oder $-CH_2$-$CHR^7$-$CH_2$- darstellen,

$R^3$ H oder $C_{1-5}$-Alkyl bedeutet,

$R^4$ H bedeutet,

$R^5$ $C_{1-4}$-Alkyl, O-$C_{1-4}$-Alkyl, O-Benzyl, $CHF_2$, $CF_3$, O-$CF_3$, Cl oder F darstellt und

$R^6$ H bedeutet,

$R^7$ $C_{1-8}$-Alkyl, $C_{3-8}$-Cycloalkyl, O-$C_{1-4}$-Alkyl, O-Benzyl, $CF_3$, Cl oder F bedeutet, wobei die Racemate der Verbindungen der Formel I,

in der $R^1$ und $R^2$ zusammen $-(CH_2)_3$- sind, $R^3$, $R^4$ und $R^6$ H bedeuten und $R^5$ $OCH_3$ ist, oder

in der $R^1$ und $R^2$ zusammen $-(CH_2)_3$- sind, $R^3$, $R^4$ und $R^6$ H bedeuten und $R^5$ O-CO-$CH_3$ ist, oder

in der $R^1$ und $R^2$ zusammen $-(CH_2)_2$- sind, $R^3$, $R^4$ und $R^6$ H bedeuten und $R^5$ OH ist, als Hydrochlorid, oder

in der $R^1$ und $R^2$ zusammen $-(CH_2)_3$- sind, $R^3$, $R^4$ und $R^6$ H bedeuten und $R^5$ OH ist, als Hydrochlorid, oder

in der $R^1$ und $R^2$ zusammen $-(CH_2)_4$- sind, $R^3$, $R^4$ und $R^6$ H bedeuten und $R^5$ OH ist, als Hydrochlorid,

ausgenommen sind, dadurch gekennzeichnet, daß man ein β-Dimethylaminoketon der Formel II

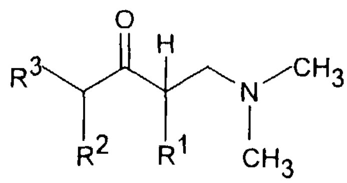

mit einer metallorganischen Verbindung der Formel III

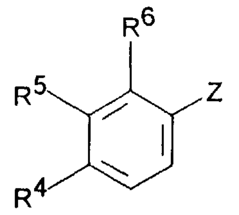

in der Z MgCl, MgBr, MgI oder Li bedeutet, zu einem tertiären Alkohol der Formel IV

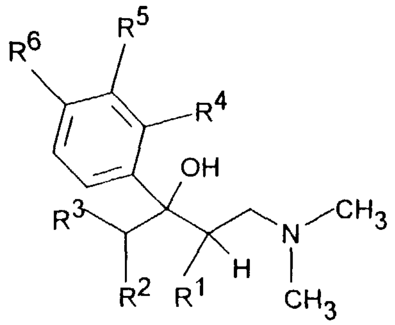

umsetzt, welcher anschließend zu einer Verbindung der Formel I dehydratisiert wird.

**6.** Verfahren zur Herstellung einer Dimethyl-(3-aryl-but-3-enyl)-aminverbindung der Formel I,

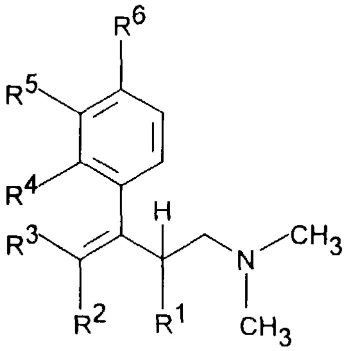

in der $R^1$ und $R^2$ zusammen $-(CH_2)_{2-4}-$, $-(CH_2)_2-CHR^7$ oder $-CH_2-CHR^7-CH_2-$ bedeuten,

$R^3$ H oder $C_{1-5}$-Alkyl bedeutet,

der Rest $R^5$ OH bedeutet und die Reste $R^4$ und $R^6$ H sind,

$R^7$ $C_{1-8}$-Alkyl, $C_{3-8}$-Cycloalkyl, O-$C_{1-4}$-Alkyl, O-Benzyl, $CF_3$, Cl oder F bedeutet

dadurch gekennzeichnet, daß man eine Verbindung der Formel I, in der der Rest $R^5$ O-$CH_3$ bedeutet und die Reste $R^4$ und $R^6$ H sind, mit Diisobutylaluminiumhydrid umsetzt oder eine Verbindung der Formel I, in der der Rest $R^5$ O-Benzyl bedeutet und die Reste $R^4$ und $R^6$ H sind, reduktiv debenzyliert.

**7.** Arzneimittel enthaltend als pharmazeutischen Wirkstoff wenigstens eine Dimethyl-(3-aryl-but-3-enyl)-aminverbindung der allgemeinen Formel I,

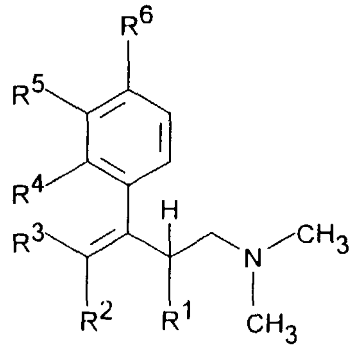

in der $R^1$ und $R^2$ zusammen $-(CH_2)_{2-4}-$, $-(CH_2)_2-CHR^7$ oder $-CH_2-CHR^7-CH_2-$ darstellen,

$R^3$ H oder $C_{1-5}$-Alkyl bedeutet,

$R^4$ H bedeutet,

$R^5$ OH, $C_{1-4}$-Alkyl, O-$C_{1-4}$-Alkyl, O-Benzyl, $CHF_2$, $CF_3$, O-$CF_3$, Cl, F oder $OR^8$ darstellt und

$R^6$ H bedeutet,

$R^7$ $C_{1-8}$-Alkyl, $C_{3-8}$-Cycloalkyl, O-$C_{1-4}$-Alkyl, O-Benzyl, $CF_3$, Cl oder F bedeutet,

$R^8$ CO-$C_{1-5}$-Alkyl, PO(O-$C_{1-4}$-Alkyl$)_2$, CO-$C_6H_4$-$R^{11}$, CO(O-$C_{1-5}$-Alkyl), CO-$CHR^{12}$-$NHR^{13}$, CO-NH-$C_6H_3$-$(R^{14})_2$ oder eine unsubstituierte oder substituierte Pyridyl-, Thienyl-, Thiazoyl- oder Phenylgruppe bedeutet,

$R^{11}$ OC(O)-$C_{1-3}$-Alkyl in ortho-Stellung oder $CH_2$-N-$(R^{15})_2$ in meta- oder para-Stellung, wobei $R^{15}$ $C_{1-4}$-Alkyl oder beide Reste $R^{15}$ zusammen mit N den 4-Morpolino-Rest bilden, bedeutet,

$R^{12}$ und $R^{13}$ gleich oder verschieden sind und

H, $C_{1-6}$-Alkyl oder $C_{3-8}$-Cycloalkyl oder $R^{12}$ und $R^{13}$ zusammen $-(CH_2)_{3-8}-$ bedeuten,

$R^{14}$ H, OH, $C_{1-7}$-Alkyl, O-$C_{1-7}$-Alkyl, Phenyl, O-Aryl, $CF_3$, Cl oder F bedeutet, mit der Maßgabe, daß die beiden Reste $R^{14}$ gleich oder verschieden sind,

mit der Maßgabe, daß die Racemate der Verbindungen der Formel I,

in der $R^1$ und $R^2$ zusammen $-(CH_2)_3-$ sind, $R^3$, $R^4$ und $R^6$ H bedeuten und $R^5$ $OCH_3$ ist
oder

in der $R^1$ und $R^2$ zusammen $-(CH_2)_3-$ sind, $R^3$, $R^4$ und $R^6$ H bedeuten und $R^5$ O-CO-$CH_3$ ist

ausgenommen sind

in Form ihrer Base und/oder ihres Salzes einer physiologisch verträglichen Säure, als Enantiomer oder Racemat und gegebenfalls weitere Wirkstoffe und/oder Hilfsstoffe.

**8.** Arzneimittel nach Anspruch 7 zur Bekämpfung von Schmerz.

**9.** Verwendung wenigstens einer Dimethyl-(3-aryl-but-3-enyl)-aminverbindung der Formel I gemäß Anspruch 7 in Form ihrer Base und/oder ihres Salzes einer physiologisch verträglichen Säure, als Enantiomer oder Racemat zur Herstellung eines Arzneimittels zur Bekämpfung von Schmerzen.